# FASCICULE DE BREVET EUROPEEN

(11) **EP 2 113 502 B1**
(45) Date de publication et mention de la délivrance du brevet: **14.11.2012**
(21) Numéro de dépôt: 09166657.8
(22) Date de dépôt: 31.01.2005
(51) Int. Cl.: C07D 333/70, C07D 307/54, C07D 333/38, C07D 307/68, C07D 209/18, C07D 279/16, C07D 215/50, C07D 209/80, C07D 405/12, A61K 31/47, A61K 31/40, A61K 31/54, A61K 31/335, A61K 31/38, C07C 251/00

(54) **Composés de type hydrazide et leur utilisation dans des compositions pharmaceutiques pour le traitement des maladies cardiovasculaires**
Verbindungen des Hydrazid-typs und deren Verwendung in pharmazeutischen Zusammensetzungen zur Behandlung cardiovaskulärer Erkrankungen
Hydrazyde type compounds and the use thereof in pharmaceutical compositions for the treatment of cardiovascular diseases

(30) Priorité: 30.01.2004 FR 0400913
(43) Date de publication de la demande: 04.11.2009
(62) Demande divisionnaire de: 05717518.4
(73) Titulaire: Clinigenetics, 30132 Caissargues (FR)
(72) Inventeur: Marguerie, Gérard, 94400, Vitry-sur-Seine (FR); Malaud, Eric, 30900, Nîmes (FR)
(74) Mandataire: Maureau, Philippe

(56) Documents cités:
- US-A- 3 829 492
- PATENT ABSTRACTS OF JAPAN vol. 1999, no. 09, 30 juillet 1999 (1999-07-30) & JP 11 106371 A (NISSHIN FLOUR MILLING CO LTD), 20 avril 1999 (1999-04-20)
- TRIVEDI ET AL.: "Synthesis and antimicrobial activity of some heterocyclic compounds" IND. J. CHEM., vol. 32B, 1993, pages 497-500, XP001181798
- GEORGIEVA ET AL.: "Isonicotinoylhydrazone Analogs of Isoniazid: Relationship between Superoxide Scavenging and Tuberculostatic Acivities" BIOCHEMISTRY (MOSCOW), vol. 67, no. 5, 2002, pages 588-591, XP001181795
- PATEL ET AL.: "Studies on Anitubercular and Antibacterial Agents: prpearation of 1-(4-Amino benzoyl)-2-benzalhydrazine and 1-[4-(phenyl-thioureido)bezoyl]-2-substitu ted benzalhydrazine" J. IND. CHEM. SOC., vol. 61, 1984, pages 718-720, XP001181932
- SHAH ET AL.: "Studies on isoniazide derivatives. Preparation and Antimicrobial Activity of 2-Aryl-3-(pyridylcarbamoyl)-5-carboxymethy l-4-thiazolidinones" J. IND. CHEM. SOC., vol. 62, no. 3, 1985, pages 255-257, XP001182001
- DAVE ET AL.: "Studies on Thiazolidinones as potential Antitubercular Compounds" J. INDIAN CHEM. SOC., vol. 63, no. 3, 1986, pages 320-322, XP001181800
- COWPER ET AL.: "Studies on Aminonitriles as Drug potentials : Aminonitries of Isoniazide" J. INST. CHEMISTS (INDIA), vol. 53, no. 7, 1981, pages 195-197, XP001181937
- GRAMMATICAKIS: "No 160. - Contribution a l'étude de l'absorption dans l?ultraviolet moyenet le visible des aryl- et aroyl-hydrazones. VII. - Nitrobenzoylhydrazones (o, m et p), 2e mémoire" BULLETIN DE LA SOCIETE CHIMIQUE DE FRANCE, no. 3, 1970, pages 933-945, XP001181938
- CHARY ET AL.: "Synthesis of 2-methyl-N-(4-oxo-2-aryl-3-thiazolidinyl)- 1,8-naphthyridine-3-carboxamides" SULFUR LETTERS, vol. 8, no. 2, 1988, pages 79-88, XP001181799
- SHILIKADZE ET AL.: BULLETIN OF THE ACADEMY OF SCIENCES OF THE GEORGIAN SSR, vol. 91, no. 1, 1978, pages 145-148, XP001182018
- SALEM: "Clinico pathological studies on some newly synthetic quinoline derivatives" J. DRUG RES. EGYPT, vol. 12, no. 1-2, 1980, pages 107-114, XP002284235
- CARIATI ET AL.: "Synthesis, structure, and Second-Order Nonlinear optical Properties of Copper(II) and Palladium(II) Acentric Complexes with N-Salicylidene-N'-aroylhydrazine Tridentate Ligands" INORGANIC CHEMISTRY, vol. 41, no. 25, 2002, pages 6597-6603, XP002284236
- KAUPPI ET AL.: "Targeting Bacterial Virulence: Inhibitors of Type III Secretion in Yersinia" CHEMISTRY & BIOLOGY, vol. 10, no. 3, mars 2003 (2003-03), pages 241-249, XP002284237

## Description

La présente invention concerne de nouveaux composés de type hydrazide et leur utilisation comme agents actifs dans des compositions pharmaceutiques destinées notamment au traitement ou à la prévention des maladies cardiovasculaires.

Malgré une recherche pharmacologique très active et de grandes avancées dans les domaines chirurgicaux, les maladies cardiovasculaires, accidents coronariens et ischémies cérébrales, demeurent la cause principale des décès et des invalidités dans le monde industrialisé. Le diabète de type II et le syndrome métabolique qui lui est associé, l'hypercholestérolémie, l'obésité définie comme une augmentation de la masse graisseuse, l'hypercholestérolémie, l'hypertriglycéridémie, et la dyslipidémie athérogène caractérisée par des profils complexes de lipoprotéines sont les facteurs de risques reconnus de ces maladies cardiovasculaires.

Ces pathologies ont en commun un désordre du métabolisme des lipoprotéines. La dyslipidémie athérogène du diabétique de type II et du syndrome métabolique, par exemple, est caractérisée par un taux élevé de triglycérides (supérieur à 150 mg/dl), un taux de lipoprotéine de haute densité cholestérol bas (HDLc inférieur à 40 mg/dl), un taux de lipoprotéine de basse densité (LDLc) variable (inférieur ou supérieur à 100 mg/dl). Dans l'hypercholestérolémie, le taux circulant du LDL cholestérol est supérieur à 130 mg/dl, le taux de triglycéride est peu modifié voire normal. L'hypertriglycéridémie très souvent associée à l'obésité est caractérisée par une très forte augmentation des triglycérides (supérieur à 200 mg/dl) qui entre dans la structure des lipoprotéines.

La complication la plus sérieuse de tous ces syndromes est l'athérothrombose. L'athérothrombose est une pathologie complexe liée à ces désordres métaboliques et dont le développement est silencieux, progressif et peut débuter très tôt dans la vie en impliquant plusieurs phases successives.

La formation d'une plaque artérielle riche en lipide est un processus lent se développant en général sur plusieurs décennies. Il s'agit d'une accumulation progressive de lipoprotéines, de macrophages spumeux, et de calcium au niveau de la paroi artérielle. Les plaques affectent la plupart des individus soumis au régime riche en graisses animales habituel des pays occidentaux industrialisés, mais il existe une forte variabilité interindividuelle de la vitesse d'évolution et de l'extension des plaques qui est due en partie à des caractéristiques génétiques.

La présence de nombreux macrophages spumeux dans la plaque la rend vulnérable et occasionne des épisodes de rupture. La rupture de la plaque d'athérosclérose et la formation d'un thrombus plaquettaire sont quant à eux des processus aigus responsables des complications sévères de la maladie : l'infarctus coronaire et cérébral et la mort subite. La sévérité de la maladie dépend donc en grande partie de la taille de la plaque, de sa stabilité et de la manière dont le thrombus est formé par la rupture de cette plaque. Ce phénomène est assez mal connu et implique souvent un état inflammatoire chronique et une réponse immunologique. A ce jour, différentes options thérapeutiques sont disponibles pour le traitement de ces maladies.

Les agents hypolipémiants comme les statines ou l'ezetimibe, ont une efficacité reconnue. Les statines sont des inhibiteurs de la 3-hydroxy-methylglutaryl coenzyme A réductase qui est directement impliquée dans la synthèse du cholestérol. Les statines réduisent efficacement le taux de cholestérol et plus modestement le taux de triglycérides. L'ezetimibe inhibe l'absorption intestinale du cholestérol. Ces molécules sont donc préconisées en prévention primaire et secondaire pour la plupart des patients dont le taux de LDLc est élevé. Les essais cliniques ont cependant démontré que le bénéfice médical des agents hypolipémiants, concernant le risque cardiovasculaire, n'est que de 30 à 35%. Leur utilisation s'accompagne quelquefois d'effets secondaires non désirés qui imposent l'arrêt du traitement. Dans de nombreux cas, des atteintes musculaires, une toxicité hépatique et des phénomènes d'intolérance sont observés.

Les fibrates ou dérivés de l'acide fibrique sont également préconisés pour le traitement des dyslipidémies athérogène. Les dyslipidémies recouvrent des patients différents aux profils lipidique complexes : faible taux de cholestérol, taux élevés de triglycérides, faibles taux de HDLc. L'utilisation des fibrates réduit le risque d'accidents cardiovasculaires de 40 % environ. Leur utilisation s'accompagne malheureusement chez de nombreux patients d'effets non désirés dus à l'intolérance, une toxicité hépatique, et des atteintes musculaires.

L'accident thrombotique qui est la conséquence de la rupture d'une plaque artérielle, est en général traité par des agents antithrombotiques comme l'acide acétylsalicylique les thienopyridines ou les thyanopyridines.

La demande de brevet JP 11/106371 décrit des dérivés acylhydrazone pouvant notamment être utilisés pour le traitement de complications diabétiques diverses.

Il existe néanmoins toujours un besoin pour de nouveaux composés capables de soigner les maladies cardiovasculaires et notamment pour traiter la croissance et la vulnérabilité d'une plaque artérielle.

La présente invention a précisément pour objet de nouveaux composés de type hydrazide utiles comme agent actif dans des compositions pharmaceutiques notamment pour le traitement ou à la prévention des maladies cardiovasculaires.

Les composés de l'invention répondent à la formule générale (I) suivante : 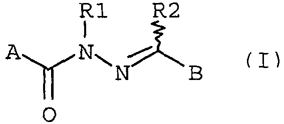 dans laquelle :
- R1 et R2 sont choisis comme étant chacun un atome d'hydrogène,
- A représente un groupement aromatique de formule (V) suivante : 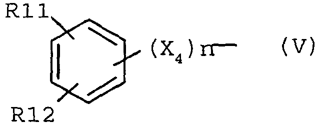 dans laquelle :
   - n est 0 ou 1
   - X₄ s'il est présent représente un groupe -CH₂- ou - OCH₂-, -CH=CH-,
   - R11 et R12, identiques ou différents, en positions *ortho, méta* ou *para* par rapport à la liaison avec -X₄- ou par rapport à la liaison avec -CO- lorsque n est 0, sont choisis parmi : un groupement alkyle inférieur à chaîne linéaire ou ramifiée de 1 à 6 atomes de carbone ou aralkyle ou un radical fluoroalkyle ayant de 1 à 6 atomes de carbone et de 3 à 7 atomes de fluor, un radical -OH, -OR13 où R13 représente un groupement alkyle inférieur à chaîne linéaire ou ramifiée de 1 à 6 atomes de carbone, un halogène et plus particulièrement du fluor et tout spécialement dans ce cas, lorsque R11 et R12 sont des atomes de fluor, ils sont en *ortho* de part et d'autre de la liaison avec -X₄- ou le reste -CO-,
   ou R12 représente un atome d'hydrogène et R11 représente un groupement sulfonamide de type -SO₂NH₂ en *para* par rapport à la liaison avec -X₄- ou le reste -CO-,
   ou encore R11 représente un atome d'hydrogène et R12 représente un groupe -Ophényl en *ortho* par rapport à la liaison avec -X₄- ou le reste -CO-,
- B représente un groupement de formule (II) suivante : 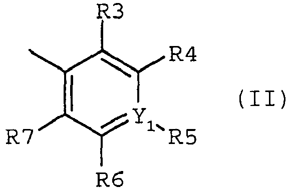 dans laquelle :
- Y₁ est un atome de carbone pour former un noyau phényle ou un atome d'azote pour former un noyau pyridine,
- R3 est un groupe -OR8 où R8 représente un radical alkyl inférieur linéaire ou ramifié de 1 à 6 carbones, un groupe amino -NH₂ ou un groupe -N(r, r') dans lequel r et r' identiques ou différents représentent un radical alkyle inférieur linéaire ou ramifié, un radical aryle, ou un hétérocycle dans lequel r et r' pris ensemble forment un hétérocycle de taille variable, de préférence en position *para,*
- R4, R5, R6 et R7, identiques ou différents, sont choisis parmi : un atome d'hydrogène, un atome d'halogène et plus particulièrement de fluor, chlore et brome, un groupe de formule -OH, -OR8 ou -OCOR9, où R8 et R9 représentent un radical alkyl inférieur linéaire ou ramifié de 1 à 6 carbones, un groupe amino -NH₂ ou un groupe -N(r, r') dans lequel r et r' identiques ou différents représentent un radical alkyle inférieur linéaire ou ramifié, un radical aryle, ou un hétérocycle dans lequel r et r' pris ensemble forment un hétérocycle de taille variable, de préférence en position para,
au moins deux des substituants R4, R5, R6 et R7 étant choisis comme étant un atome d'hydrogène.

Du fait de la fonction hydrazide au niveau de la double liaison N=C et de la signification des groupes B et R2, les composés de l'invention de formule (I) peuvent se présenter sous des formes géométriques dites (E) ou (Z) existant soit en équilibre soit sous une forme unique préférentiellement (E) :
- forme (E) dans laquelle les groupements ACONR1 et B sont de part et d'autre de la fonction imine N=C, dite forme *trans,* ou
- forme (Z) dans laquelle les groupements ACONR1 et B sont d'un même côté de la fonction imine N=C, dite forme cris.

Des composés préférés sont ceux de formule (I) dans laquelle dans laquelle Y₁ est un atome de carbone.

L'invention se rapporte également aux composés suivants :
- (4-diméthylamino)-N'-[(1E)-(2-hydroxy-4,6-diméthoxyphényl)méthylène]benzohydrazide (désigné CGP02-04),
- 2-phenéthylbenzoïque acide (2-hydroxy-4,6-diméthoxy-benzylidène)-hydrazide (désigné CGP02-05),
- N-[3-2-hydroxy-4,6-dimethoxy-benzylidène-hydrazinocarbonyl)-phényl]-propionamide (désigné CGP02-06),
- (3-chloro-phénoxy)-acétique acide (2-hydroxy-4,6-diméthoxybenzylidène)-hydrazide (CGP02-09),
- 2-phénoxy-benzoïque acide (2-hydroxy-4,6-diméthoxybenzylidène)-hydrazide (désigné CGP02-11),
- 2,6-difluorobenzoïque acide(2-hydroxy-4,6-diméthoxybenzylidène)-hydrazide (désigné CGP02-13),
- 4-trifluorométhylbenzoïque acide (2-hydroxy-4,6-diméthoxy-benzylidène)-hydrazide (désigné CGP02-16),
- 3,4-diméthoxybenzoïque acide (4-diéthylamino-2-hydroxy-benzylidène)-hydrazide (désigné CGP02-17).

L'invention concerne aussi, quand cela est possible, les sels des composés ci-dessus avec des acides de type pharmaceutique tolérés physiologiquement.

A titre d'exemple de sels pharmaceutiques physiologiquement acceptables, on peut citer de manière non limitative les sels d'acide acétique, chlorhydrique, cinnamique, citrique, formique, hydrobromique, hydroiodique, hydrofluorique, malonique, méthanesulfonique, oxalique, picrique, maléique, lactique, nicotinique, plénylacétique, phosphorique, succinique, tartrique, les sels d'ammonium, de diéthylamine, de pipérazine, de nicotinamide, d'urée, de sodium, de potassium, de calcium, de magnésium, de zinc, de lithium, de méthylamino, de diméthylamino, de triméthylamino, de tris(hydroxyméthyl)aminométhane.

L'invention concerne les compositions pharmaceutiques pour l'homme ou l'animal comprenant à titre d'agent actif au moins un des composés décrits ci-dessus ou leur sel pharmaceutiquement acceptable.

En effet, ces composés sont utiles pour le traitement de l'athérosclérose et de la resténose artérielle. Ils ont la propriété de diminuer la prise de poids due à une accumulation de graisse abdominale, de diminuer l'augmentation du taux de cholestérol total et du cholestérol libre et le dépôt de triglycérides au niveau de la paroi artérielle et de réduire l'accumulation de macrophages au niveau des plaques athéromateuses. Ces composés ont en particulier la propriété d'inhiber la formation de cellules macrophages spumeuses en inhibant l'accumulation de vésicules lipidiques intra-cellulaire. Par extension, ces molécules sont donc capables de traiter l'obésité, le diabète de type II, l'ischémie cérébrale et les stéatoses hépatiques, en bloquant l'accumulation de vésicules lipidiques dans des cellules telles que l'hépatocyte, la cellule du muscle lisse, l'adipocyte et la cellule endothéliale.

Ces composés sont donc utiles comme agents actifs dans des méthodes ou des compositions pharmaceutiques pour le traitement et éventuellement la prévention de toutes les maladies associées à des désordres du métabolisme des lipides. A ce titre, on peut citer entre autres, l'hypercholestérolémie, l'hypertriglycéridémie , la dyslipoproteinémie, la chylomicronémie, la lipodistrophie, l'hyperglycémie, ainsi que les pathologies associées à ces dysfonctionnements : l'athérosclérose, l'obésité, le diabète de type II, et la résistance à l'insuline, l'insuffisance cardiaque et l'ischémie cérébrale (stroke).

Par ailleurs, ces composés ayant la propriété de réduire le rétrécissement de la paroi artérielle, ils sont utiles comme agents actifs dans des méthodes ou des compositions pharmaceutiques pour le traitement et éventuellement la prévention de la resténose.

Les compositions pharmaceutiques selon l'invention comprennent des quantités suffisantes d'au moins un composé décrit précédemment.

Sur la base des résultats obtenus *in vivo* et présentés dans la partie expérimentale ci-après, les compositions de l'invention peuvent être administrées dans le cadre d'un traitement en une plusieurs doses de 0,01 à 500 milligrammes par kilogramme de poids du corps par jour d'un ou plusieurs composés de l'invention.

La formulation des compositions pharmaceutiques selon l'invention est du type généralement utilisée dans le domaine pharmaceutique.

A titre d'exemple, il peut s'agir de vecteurs pharmaceutiques tels que, par exemple, des sels ou des électrolytes, des sels de l'acide scorbique, de l'eau ou des solutions tamponnées, des solutions colloïdales, des substances à base de cellulose, du polyéthylène glycol, des polyacrylates, des cires, des protéines, ou tout autre substance capable de dissoudre ou de rendre le composé actif disponible pour une action thérapeutique.

Les compositions de la présente invention peuvent être administrées sous forme injectable ou par voie orale, parentérale, nasale sous forme de spray, rectale ou vaginale, par l'implantation de réservoir ou dispensateurs ou sous tout autre forme galénique en usage dans le domaine pharmaceutique.

Les formes injectables de ces compositions peuvent être des suspensions aqueuses ou oléagineuses. Ces suspensions peuvent être formulées selon tout procédé en usage dans ce domaine en utilisant des solvants ou des diluants non toxiques comme par exemple le 1,3-butanediol. Parmi les solvants acceptables, il est possible d'utiliser de l'eau, des solutions tamponnées, des solutions de Ringer, ou des solutions isotoniques de sels. D'autres diluants acceptables peuvent être constitués de mono ou di-glycérides synthétiques, des alcools à longue chaîne, ou des dispersants tels que la carboxymethyl cellulose ou tout autre diluant ou émulsifiant utilisés dans la formulation de suspension pharmaceutique.

Les compositions pharmaceutiques de la présente invention administrées par voie orale, peuvent être sous forme de capsule, de cachet ou de suspensions aqueuse ou sous forme d'émulsion. Ces formulations peuvent éventuellement contenir des composés chimiques destinés à adoucir ou améliorer le goût.

Les compositions pharmaceutiques de la présente invention peuvent être administrées sous forme de suppositoire en mélangeant le produit avec un excipient non irritant, non allergique, solide à la température ambiante et liquide à la température rectale de manière à libérer le composé actif. De telles formulations peuvent utiliser par exemple de la cire d'abeille, des polyéthylèneglycols ou du beurre de cacao.

Ces compositions pharmaceutiques peuvent également comprendre une combinaison d'un plusieurs composés de l'invention avec une ou plusieurs autres molécules thérapeutiques. Ces molécules peuvent être par exemple des agents hypolipémiant réduisant la synthèse du cholestérol comme les « statines », des inhibiteurs de l'enzyme de conversion de l'angiotensine II comme par exemple le Losartan, des anticalciques, des antithrombotiques, des bêta-bloquants, des inhibiteurs des membres de la famille des récepteurs activés des proliférateurs du péroxisome (famille des PPAR), des inhibiteurs de la synthèse ou du métabolisme des triglycérides comme les Fénofibrate, des agents capables d'augmenter la résistance à l'insuline comme les Troglitazone ou les Pioglitazone et d'une manière générale, toute autre molécule capable d'améliorer les performances pharmacologiques des composés décrits dans la présente invention.

L'invention concerne également l'utilisation d'un composé selon l'invention pour la préparation d'une composition pharmaceutique selon l'invention.

L'invention concerne aussi la préparation des composés de formule (I) et des compositions pharmaceutiques contenant comme principe actif au moins un desdits composés.

Les composés de formule (I) peuvent être préparés selon les techniques connues de l'homme de métier. La présente invention décrit à cet égard une voie de synthèse générale qui est illustrée par le schéma ci-dessus et dans l'exemple de mode opératoire qui suit où les composés de départ sont obtenus dans le commerce ou peuvent être synthétisés selon des procédés habituels connus de l'homme de métier et décrits dans les livres classiques de chimie organique ("Advanced Organic Chemistry" de M. B. Smith & J. March, Ed. John Wiley & Sons, "Handbook of Heterocyclic Chemistry" de A. R. Katritzky, Ed. Pergamon, et "Heterocyclic Chemistry" de J. A. Joule et K. Mills, Ed. Blackwell Science).

Il est entendu que l'invention n'est pas limitée à une voie de synthèse particulière, et s'étend à d'autres procédés permettant la production des composés de formule (I). A titre d'exemple, les composés de formule (I) peuvent ainsi être préparés soit en phase liquide soit en phase parallèle sur support solide. Les méthodes ci-dessous sont données à titre non limitatif, et tous autres procédés permettant de créer des doubles liaisons de type imines N=C substituées peuvent êtres utilisés pour préparer les composés de l'invention.

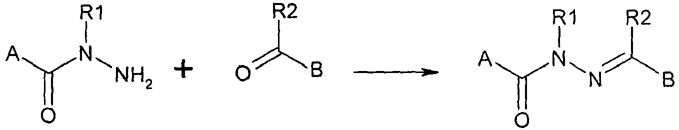

Dans le schéma ci-dessus, R1, R2, A et B ont la même signification que précédemment.

Selon le schéma ci-dessus, les composés de l'invention de formule (I) sont directement préparés par une réaction de condensation entre d'une part la matière première désignée carbo-hydrazide représentée par la formule A-CO-NR1-NH2 et un aldéhyde ou une cétone représentée par la formule R2-COB, pour lesquels les groupes A et R1 d'une part et B et R2 d'autre par ont respectivement les significations décrites pour les formules (II) à (VIII). Ces matières premières de départ mises en jeu sont commerciales et peuvent être acquises auprès de sociétés de chimie à façon telles que Maybridge (Grande-Bretagne) ou Pfaltz-Bauer (USA), ce choix de sociétés n'étant pas exclusif.

Cette réaction de condensation est conduite préférentiellement en atmosphère inerte, entre 0°C et 50°C de préférence à température ambiante en présence d'une base organique amine tertiaire de préférence la base de Hünig di-isopropyléthylamine DIEA, dans un solvant dipolaire aprotique de préférence le diméthylformamide DMF anhydre ou dans l'éthanol à reflux pendant 6 à 8 heures. Le monitoring de l'avancement de la réaction est réalisé par analyse HPLC permettant de contrôler le temps de réaction préférentiellement inférieur à 24 h.

D'autres avantages et caractéristiques de l'invention apparaîtront des exemples qui suivent et dans lesquels il sera fait référence aux dessins en annexe où :

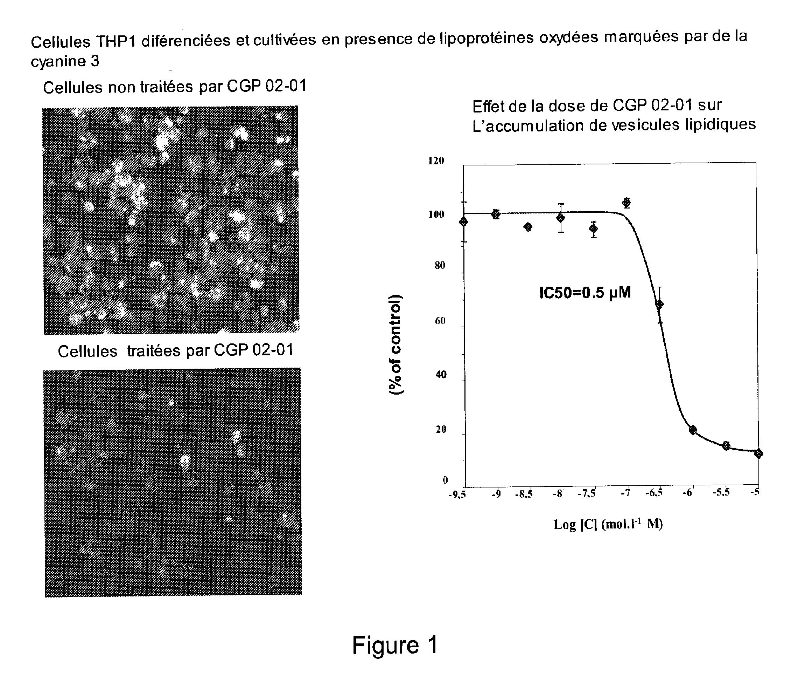

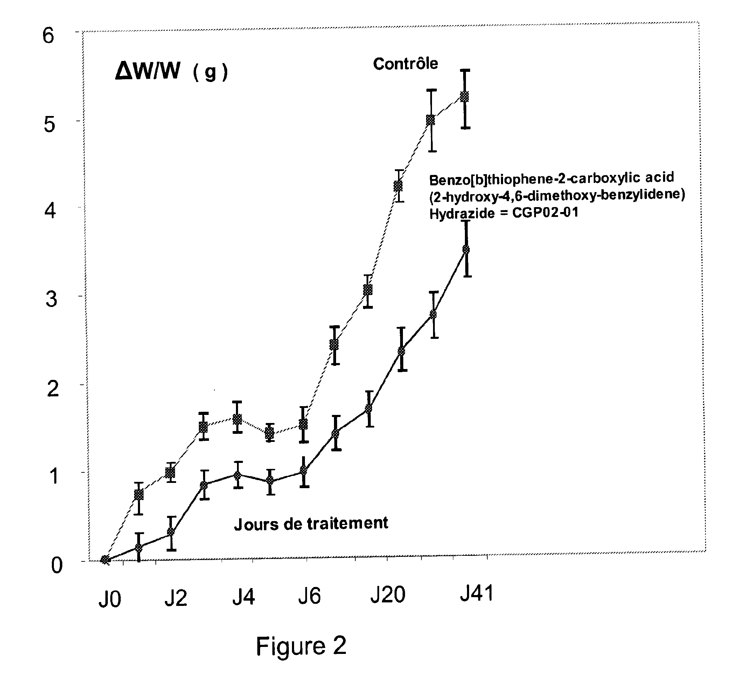

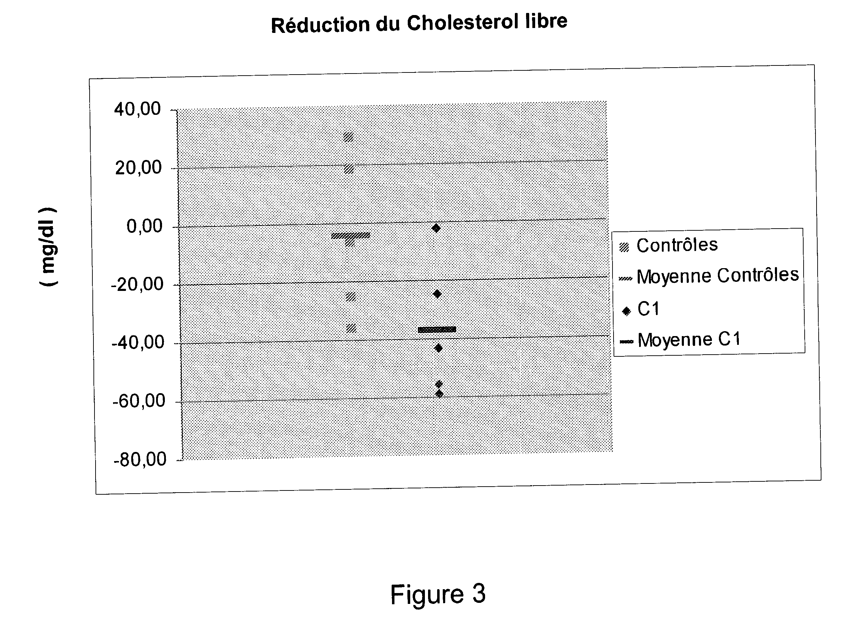

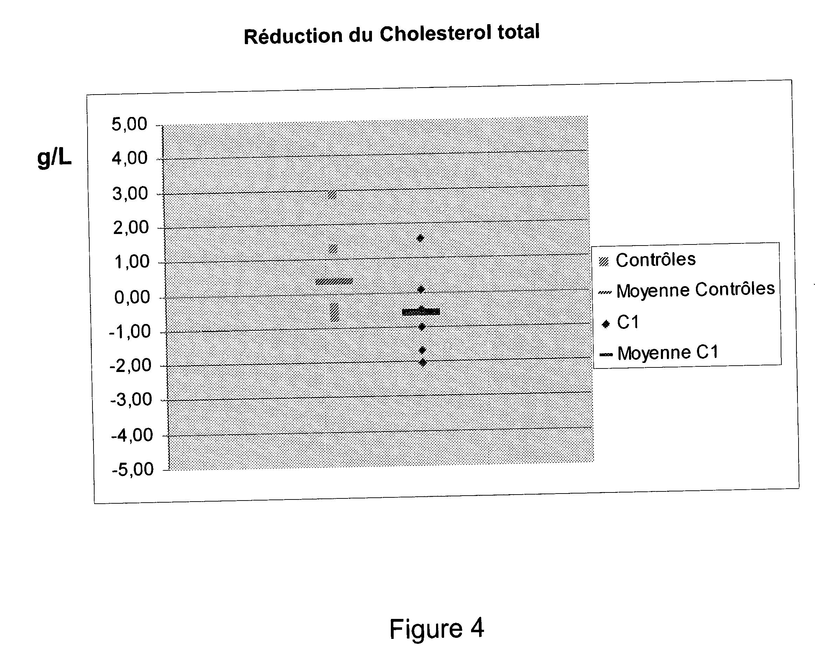

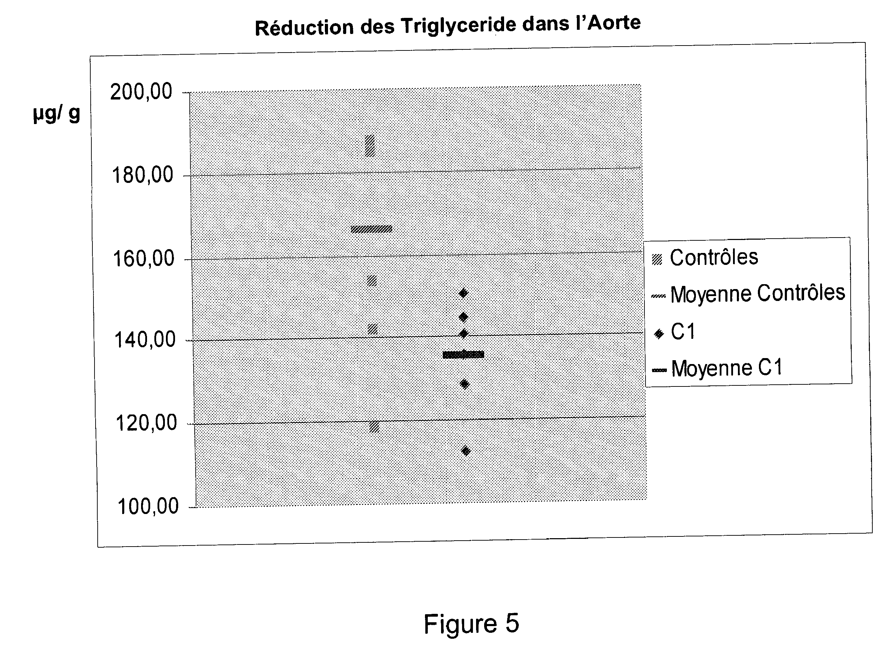

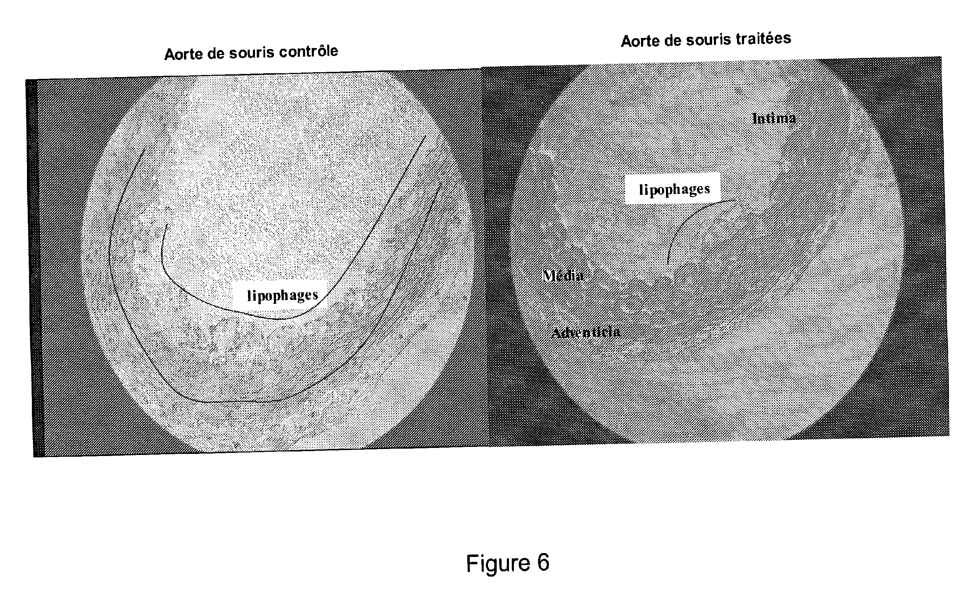

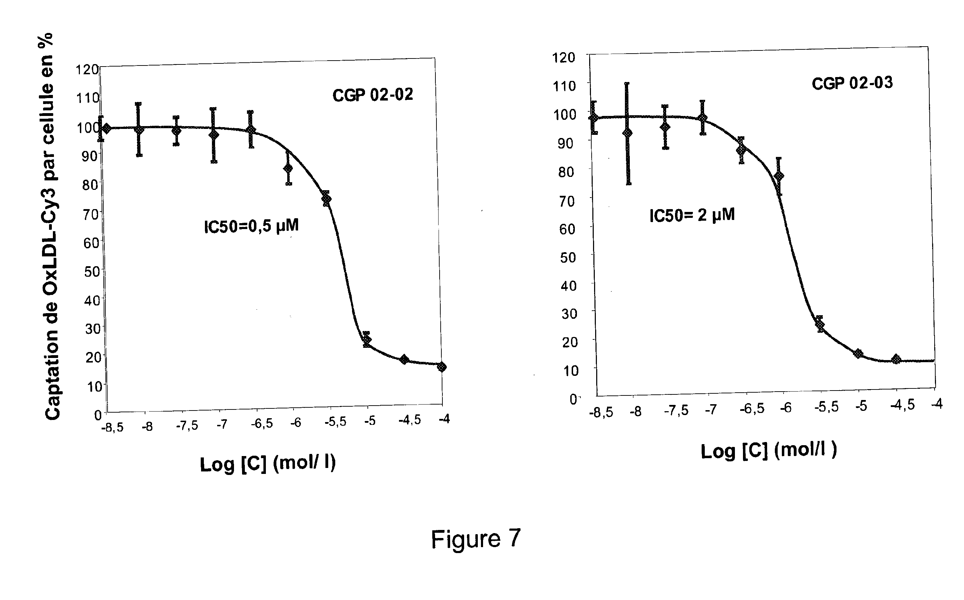

- La figure 1 montre l'inhibition de la formation de cellules spumeuses par les composés CGP 02-02 et CGP 02-03. Les cellules THP1 différenciées sont cultivées en présence de lipoprotéines oxydées (3 µg/ml oxLDL) marquées par de la cyanine 3 pendant 24 heures à 37°C. Les cellules sont traitées par les composés CGP 02-02 et CGP 02-03 à différentes concentrations. Les conditions sont identiques à celles de la figure 1.

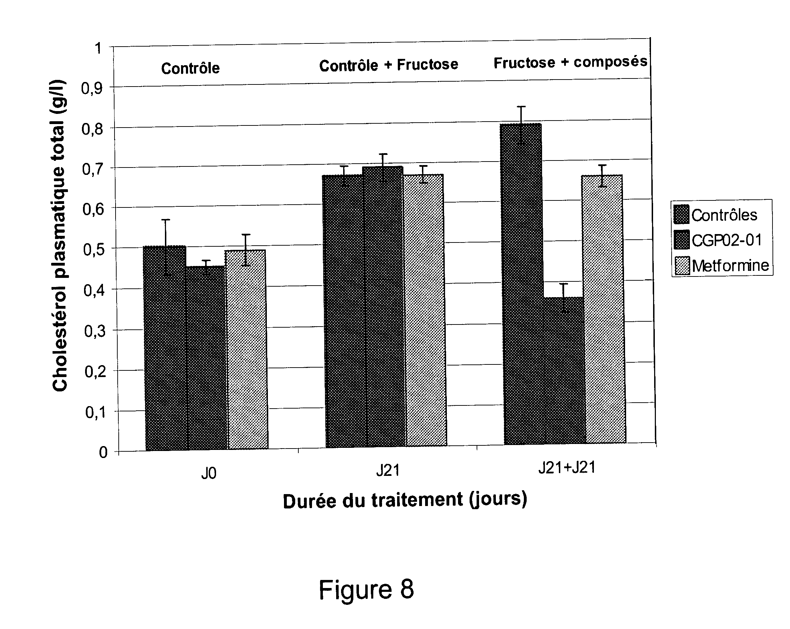

- La figure 8 représente l'effet du composé CGP 02-01 administré par voie orale (50 mg/kg) sur le taux plasmatique de cholestérol total (g/L) après 3 semaines de traitement d'un modèle de rat (n=12) ,soumis à un régime riche en fructose (10 %). La metformine a été injectée à la même dose pour servir de témoin standard dans ce modèle animal.

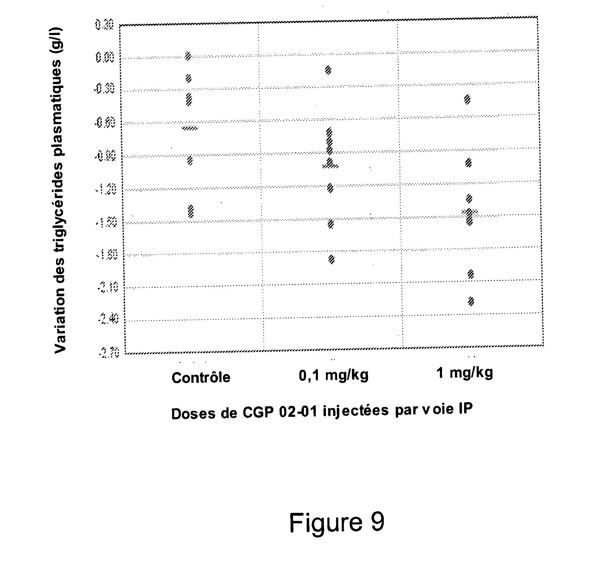

- La figure 9 illustre les variations du taux plasmatique de triglycérides (g/L) chez une souris ApoE -/soumise à un régime riche en cholestérol et traitée pendant 3 mois avec le composé CGP 02-01.

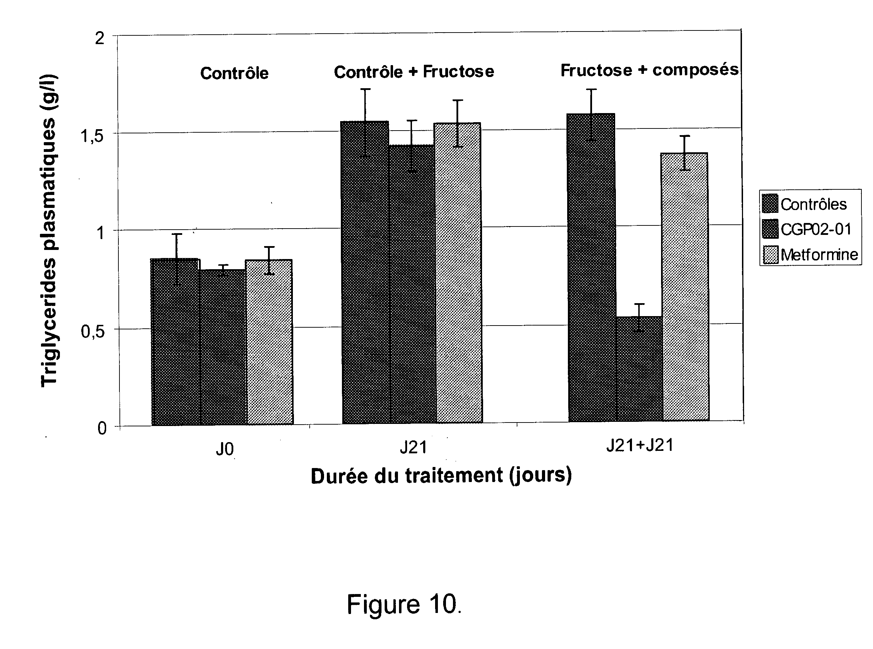

- La figure 10 montre l'effet du composé CGP 02-01 administré par voie orale (50 mg/kg) sur le taux plasmatique de triglycérides (g/L) après 3 semaines de traitement sur un modèle de rat (n=12) ,soumis à un régime riche en fructose (10 %). Les rats sont nourris quotidiennement avec un régime contenant 10% de fructose pendant 3 semaines. Le régime est ensuite maintenu en ajoutant le composé CGP 02-01 pendant 3 semaines. Chaque rat est analysé séparément. La metformine a été administrée à la même dose pour servir de témoin standard dans ce modèle animal

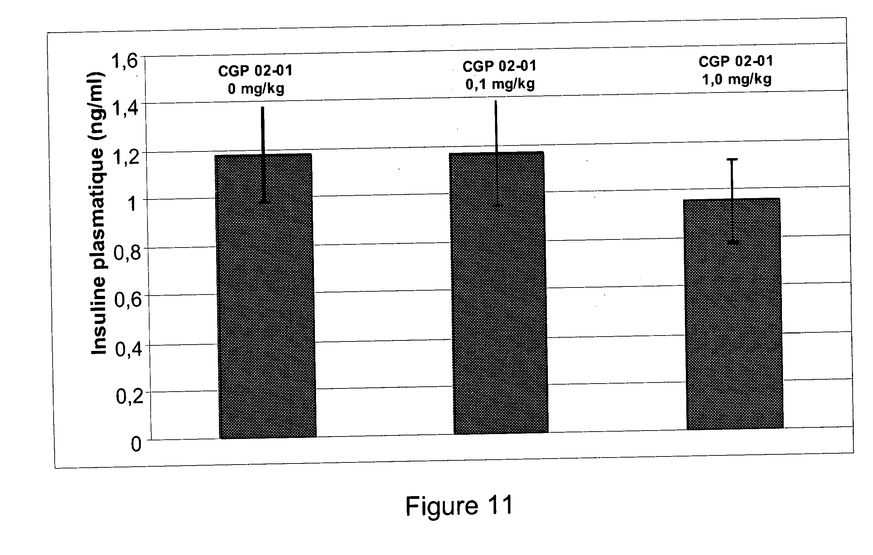

- La figure 11 représente l'effet du composé CGP 02-01 injecté par voie IP sur le taux plasmatique d'insuline (ng/mL) de souris ApoE -/- soumises à un régime riche en cholestérol. Les souris sont traitées pendant 3 mois.

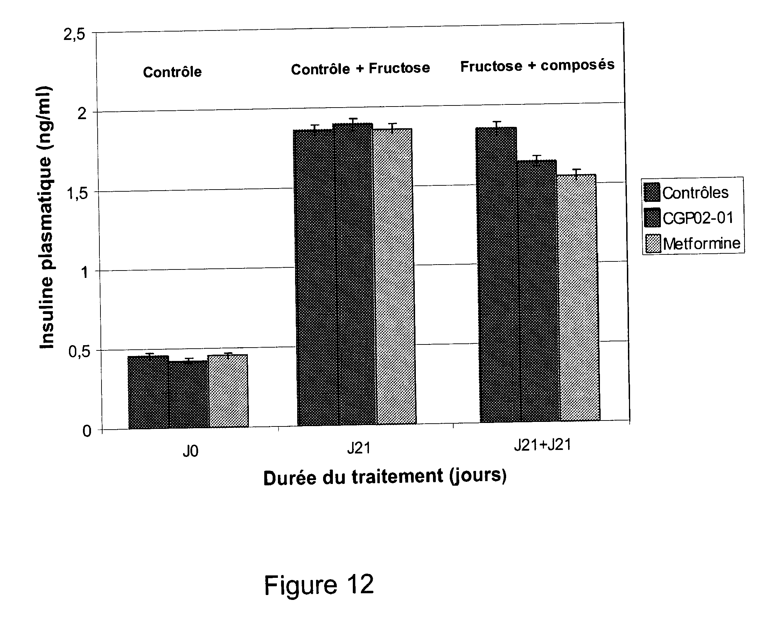

- La figure 12 montre l'effet du composé CGP 02-01 administré par voie orale (50 mg/kg) sur le taux plasmatique d'insuline (ng/mL) après 3 semaines de traitement sur un modèle de rat (n=12), soumis à un régime riche en fructose (10 %). Les rats sont nourris quotidiennement avec un régime contenant 10% de fructose pendant 3 semaines. Le régime est ensuite maintenu en ajoutant le composé CGP 02-01 pendant 3 semaines. Chaque rat est analysé séparément. La metformine a été administrée à la même dose pour servir de témoin standard dans ce modèle animal.

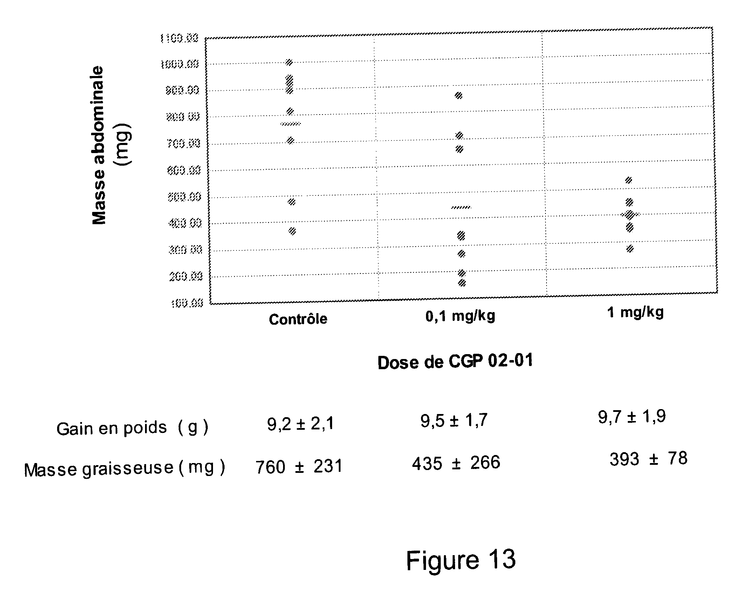

- La figure 13 montre l'effet du composé CGP 02-01 injecté par voie IP à différentes doses sur l'obésité abdominale de souris ApoE -/- soumises à un régime riche en cholestérol. Les souris sont traitées pendant 3 mois.

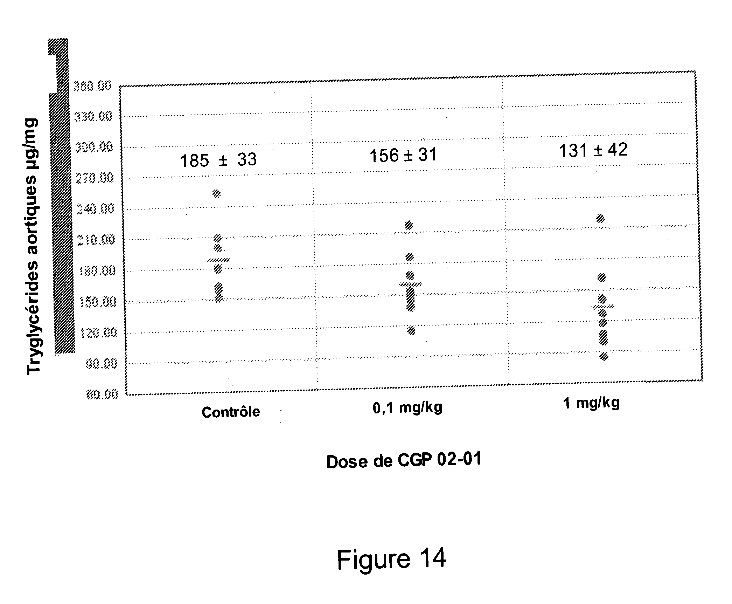

- La figure 14 montre l'effet du composé CGP 02-01 injecté par voie IP à différentes doses sur le dépôt de triglycérides dans la paroi aortique de souris ApoE -/soumises à un régime riche en cholestérol. Les souris sont traitées pendant 3 mois.

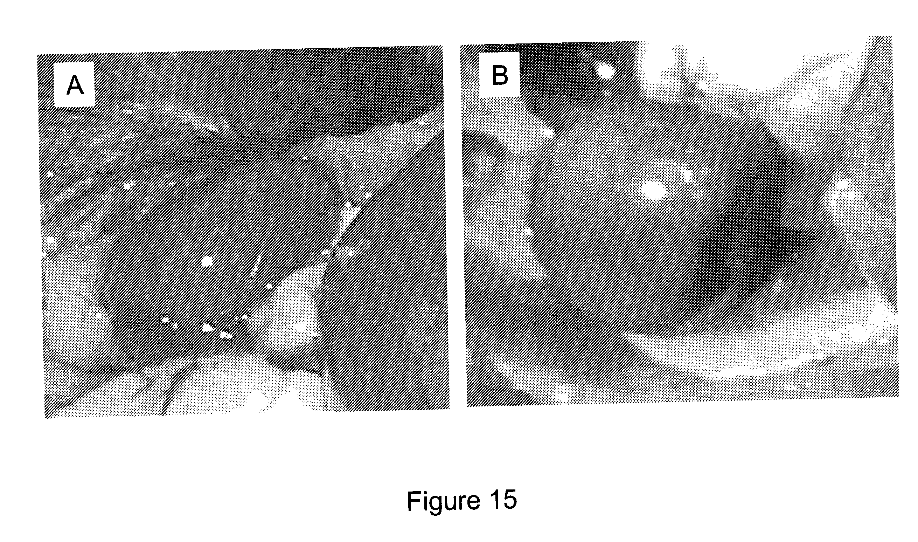

- La figure 15 montre que lorsque des souris ApoE -/sont soumises à un régime normal ou riche en cholestérol, elles développent une ischémie coronarienne illustrée par la présence d'une micro-embolisation des microvaisseaux coronariens (figure A). Lorsque le composé CGP 02-01 est injecté à ces souris, ces lésions cardiaques sont considérablement réduites (figure B).

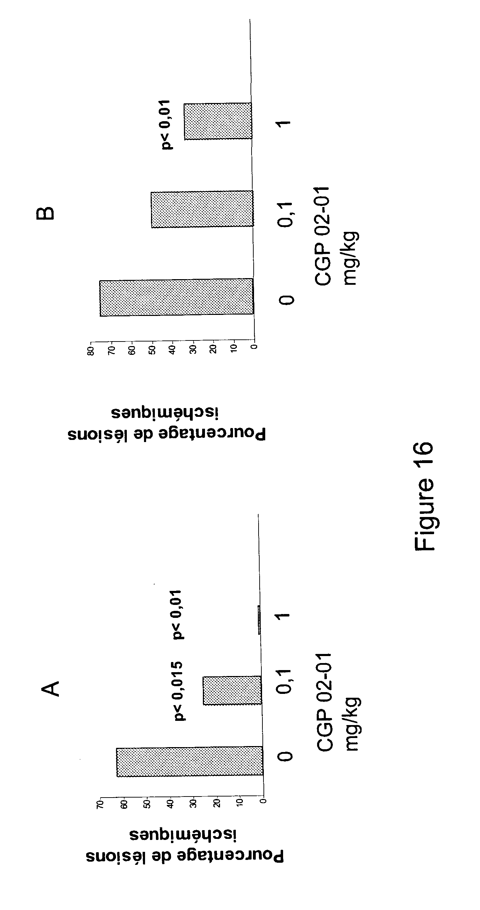

- La figure 16 illustre l'effet dose du composé CGP 02-01 sur les lésions coronariennes de souris ApoE -/soumises à un régime normal (figure A) et à un régime riche en cholestérol (figure B).

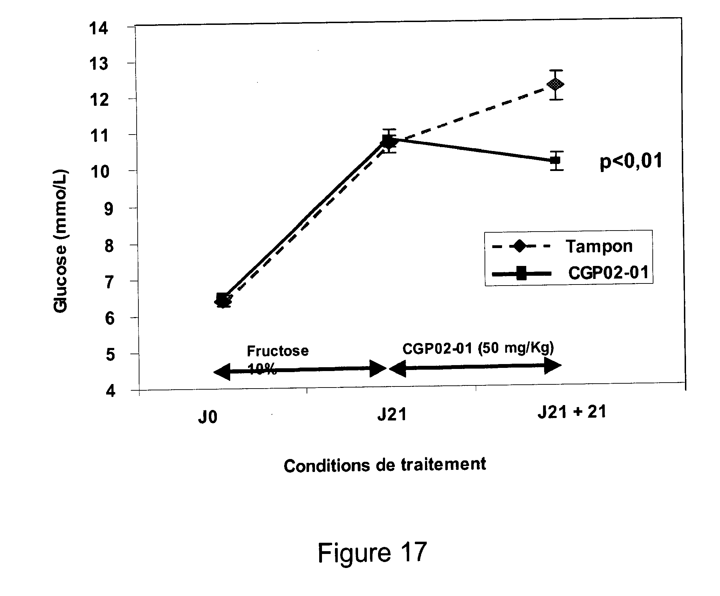

- La figure 17 illustre l'effet du composé CGP 02-01 sur l'augmentation de la glycémie chez des rats soumis à un régime riche en fructose (10%). Le régime est maintenu pendant 21 jours. Le composé est administré par voie orale suite à la période de diète fructose. La figure illustre l'effet stabilisant du composé.

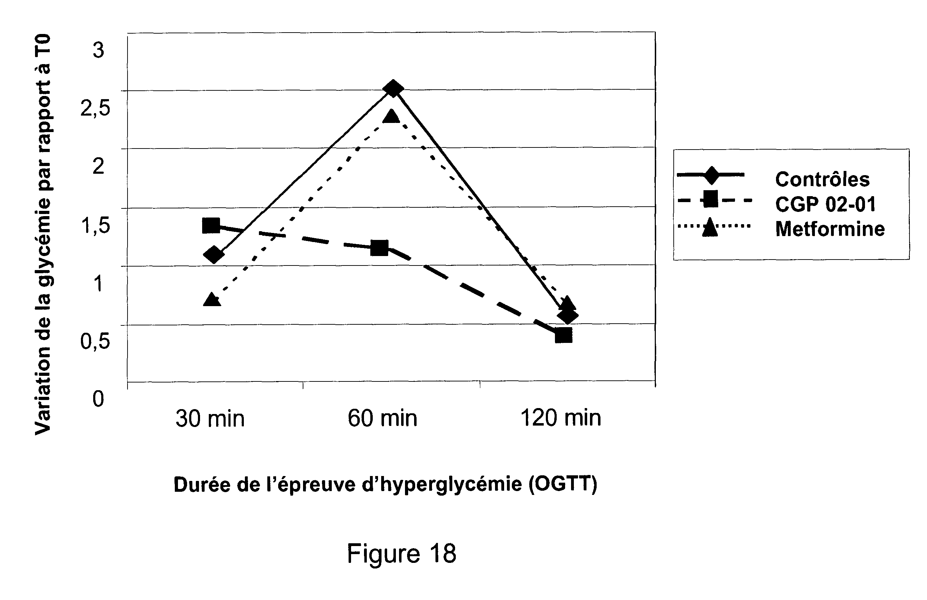

- La figure 18 illustre l'effet bénéfique du composé CGP 02-01 sur la tolérance au glucose chez des rats soumis à un régime riche en fructose (10%). Le composé est administré par voie orale, au bout du 14éme jour une dose unique supplémentaire de 2 g/kg de glucose est administrée. La glycémie est mesurée à 30, 60 et 120 minutes après ce choc glycémique.

### Exemple 1 : Cultures cellulaires.

Plusieurs lignées de cellules permanentes peuvent être utilisées pour démontrer l'effet des composés de l'invention sur la fixation et l'accumulation de lipides dans des vésicules intracellulaires. Ces cellules peuvent incorporer des lipoprotéines, des lipoprotéines modifiées, par exemple oxydées ou acétylées, des triglycérides ou des chylomicrons. Ces cellules ont la capacité de se transformer en cellules spumeuses et peuvent ainsi présenter un phénotype athérogène. Il est possible d'utiliser, à titre d'exemple, les cellules THP1, U937, KG1 ou tout autre cellule pouvant être activée et différentiée en macrophage, cellule endothéliale, cellule du muscle lisse, hépatocyte ou adipocyte puis mise en culture en présence de milieu contenant des lipoprotéines.

D'autres types de cellules ayant été génétiquement modifiées pour exprimer des récepteurs membranaires spécifiques de la captation de lipoprotéines ou des acides gras peuvent également être utilisées. Ces récepteurs membranaires peuvent faire partie de la famille des molécules scavenger contenant des protéines telles que SRAI, SRAII, SRBI, CD36 ou des membres de la famille des récepteurs des acides gras (FABP) par exemple.

A titre d'exemples, on peut citer plus particulièrement des cellules du type des cellules THP1 différenciées sous l'action de phorbol 12-myristate-13-acetate ( PMA ) à une concentration de 10⁻⁷ M, ont été utilisées pour mesurer la formation et l'accumulation de vésicules lipidiques observées au cours de la formation de macrophage spumeux en présence ou en absence du composé CGP02-01.

Les cellules sont cultivées dans des plaques à 96 puits, à une densité de 1, 2 ou 5 x 10⁵ cellules par ml en milieu RPMI-1640 ou en milieu MEM contenant 1%, 2%, 5 % ou 10% de sérum de veau foetal (SVF), 100 Unité / ml de pénicilline, 100 µg/ml de streptomycine, 200 mM de L-Glutamine à 37°C en incubateur à CO2. Le milieu de culture peut être remplacé tous les deux jours.

Dans le présent exemple, l'accumulation de vésicules lipidiques au sein de la cellule a été mesurée en utilisant des cellules THP1 après fixation par la paraformaldehyde en milieu PBS à l'aide d'une solution contenant un marqueur fluorescent de type Oil Red O pour visualiser les vésicules. L'image des cellules riches en vésicules a été analysée à l'aide d'un microscope équipé d'une caméra CCD et des logiciels nécessaires à l'analyse.

Les cellules THP-1 (5.10⁵ cellules/ml) (ECACC) étaient maintenues et cultivées dans du milieu RPMI-1640 contenant 10% de sérum de veaux foetal (FBS), 200 mM de L-Glutamine, 100 Unités/ml de pénicilline et 100 µg/ml de Streptomycine (Invitrogen-Life Technologies) à 37°C, dans un incubateur à 5% CO₂. Le milieu était remplacé tous les 2-3 jours.

Pour induire la différentiation des THP-1, 1.25 10⁵ cellules/puits étaient déposées dans des puits d'une plaque de culture 96 puits, dans leur milieu de culture contenant 10⁻⁷ M de phorbol 12-myristate-13-acetate (Sigma), pendant 24 heures à 37°C, 5% CO2. Les THP-1 différenciées étaient alors incubées avec des LDLox couplées à la cyanine-3 (1.5 µg/ml) en présence ou en absence de la molécule CGP02-01 (concentrations comprises entre 10⁻⁵M et 3.16 10⁻¹⁰M) pendant 24 heures à 37°C, 5% CO₂. Après fixation des cellules au paraformaldéhyde 4%, les noyaux étaient marqués au Hoechst 33342 (10 µg/ml) pendant 20 minutes, à température ambiante. Après deux lavages, 16 images/puits du signal liée à la cyanine-3 et au Hoechst 33342 étaient prises en utilisant un microscope à fluorescence couplé à une caméra CCD. Chaque image était analysée et quantifiée avec le logiciel MetaMorph (Universal Imaging).

Le tableau 1 ci-dessous rapporte le pourcentage observé pour l'inhibition de la captation et de l'accumulation sous forme de vésicules lipidiques, de lipoprotéines marquées à la cyanine 3, par des cellules exprimant le scavenger CD36. Les cellules ont été incubées en présence de chacune des molécules constituant cette famille à une concentration finale et identique pour chaque molécule de 2,5 µM.

**Tableau 1**

| Molécules | Inhibition de l'accumulation des LDL ox en ( % ) |
|---|---|
| CGP02-04 | 61 |
| CGP02-05 | 77 |
| CGP02-06 | 71 |
| CGP02-09 | 53 |
| CGP02-11 | 44 |
| CGP02-13 | 68 |
| CGP02-16 | 77 |
| CGP02-17 | 88 |

Le tableau de l'annexe 1 donne les structures de composés de l'invention ainsi que leur code par rapport au tableau 1 ci-dessus ainsi que leur pourcentage d'inhibition à une concentration de 25 microM sur les cellules THP1 pendant 24 heures.

| **Structure** | **Composés** | **Inhibition à 2.5 µM sur les cellules THP-1 (24 heures)** |
|---|---|---|
| 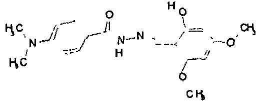 | CGP02-04 | 61% |
| 4-(dimethylamino)-N'-[(1E)-(2-hydroxy-4,6--dimethoxyphenyl)methylene] benzohydrazide | | |
| 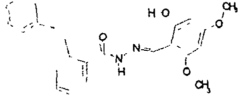 | CGP02-05 | 77% |
| 2-phenethyl-benzoic acid (2-hydroxy-4,6-dimethoxy-benzylidene)-hydrazide | | |
| 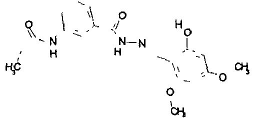 | CGP02-06 | 71% |
| N-[3-(2-Hydroxy-4,6-dimethoxy-benzylidene-hydrazinocarbonyl)-phenyl]-proplonamide | | |
| 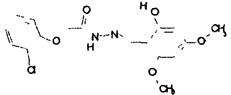 | CGP02-09 | 53% |
| (2-Chloro-phenoxy)-acetic acid (2-hydroxy-4,6-dimethoxy-benzylidene)-hydrazide | | |
| 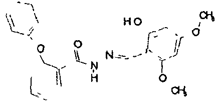 | CGP02-11 | 44% |
| 2-Phenoxy-benzoic acid (2-hydroxy-4,6-dimethoxy-benzylidene)-hydrazide | | |
| 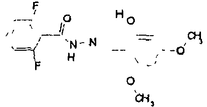 | CGP02-13 | 68% |
| 2,6-Difluoro-benzoic acid (2-hydroxy-4,6-dimethoxy-benzyhdene)-hydrazide | | |
| 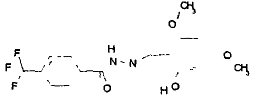 | CGP02-16 | 77% |
| 4-Trifluoromethyl-benzoic acid (2-hydroxy-4,6-dimethoxy-benzylidene)-hydrazide | | |
| 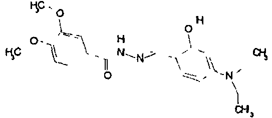 | CGP02-17 | 88% |
| 3,4-Dimethoxy-benzoic acid (4-diethylamino-2-hydroxy-benzylidene)-hydrazide | | |

## Revendications

1. Un composé de formule générale (I) suivante : 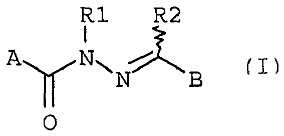 dans laquelle :
- R1 et R2 sont choisis comme étant chacun un atome d'hydrogène,
- A représente un groupement de formule (V) suivante : 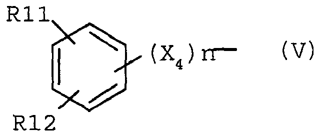 dans laquelle :
- n est 0 ou 1
- X₄ s'il est présent représente un groupe -CH₂- ou - OCH₂-, -CH=CH-,
- R11 et R12, identiques ou différents, en positions *ortho, méta* ou *para* par rapport à la liaison avec -X₄- ou par rapport à la liaison avec -CO- lorsque *n* est 0, sont choisis parmi : un groupement alkyle inférieur à chaîne linéaire ou ramifiée de 1 à 6 atomes de carbone ou aralkyle ou un radical fluoroalkyle ayant de 1 à 6 atomes de carbone et de 3 à 7 atomes de fluor, un radical -OH, -OR13 où R13 représente un groupement alkyle inférieur à chaîne linéaire ou ramifiée de 1 à 6 atomes de carbone, un halogène et plus particulièrement du fluor et tout spécialement dans ce cas, lorsque R11 et R12 sont des atomes de fluor, ils sont en *ortho* de part et d'autre de la liaison avec -X₄- ou le reste -CO-,
ou R12 représente un atome d'hydrogène et R11 représente un groupement sulfonamide de type -SO₂NH₂ en *para* par rapport à la liaison avec -X₄- ou le reste -CO-,
ou encore R11 représente un atome d'hydrogène et R12 représente un groupe -Ophényl en *ortho* par rapport à la liaison avec -X₄- ou le reste -CO-,
- B représente un groupement de formule (II) suivante : 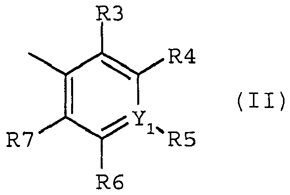
dans laquelle :
- Y₁ est un atome de carbone pour former un noyau phényle ou un atome d'azote pour former un noyau pyridine,
- R3 est un groupe -OR8 où R8 représente un radical alkyl inférieur linéaire ou ramifié de 1 à 6 carbones, un groupe amino -NH₂ ou un groupe -N(r, r') dans lequel r et r' identiques ou différents représentent un radical alkyle inférieur linéaire ou ramifié, un radical aryle, ou un hétérocycle dans lequel r et r' pris ensemble forment un hétérocycle de taille variable, de préférence en position *para,*
- R4, R5, R6 et R7, identiques ou différents, sont choisis parmi : un atome d'hydrogène, un atome d'halogène et plus particulièrement de fluor, chlore et brome, un groupe de formule -OH, -OR8 ou -OCOR9, où R8 et R9 représentent un radical alkyl inférieur linéaire ou ramifié de 1 à 6 carbones, un groupe amino -NH₂ ou un groupe -N(r, r') dans lequel r et r' identiques ou différents représentent un radical alkyle inférieur linéaire ou ramifié, un radical aryle, ou un hétérocycle dans lequel r et r' pris ensemble forment un hétérocycle de taille variable, de préférence en position *para*,
au moins deux des substituants R4, R5, R6 et R7 étant choisis comme étant un atome d'hydrogène.

2. Composé de formule (I) selon la revendication 1, **caractérisé en ce que** Y₁ est un atome de carbone.

3. (4-diméthylamino)-N'-[(1E)-(2-hydroxy-4,6 diméthoxyphényl) méthylène]benzohydrazide,
2-phenéthylbenzoïque acide (2-hydroxy-4,6-diméthoxy-benzylidène)-hydrazide,
N-[3-2-hydroxy-4,6-dimethoxy-benzylidène-hydrazinocarbonyl)-phényl]-propionamide,
(3-chloro-phénoxy)-acétique acide (2-hydroxy-4,6-diméthoxybenzylidène)-hydrazide,
2-phénoxy-benzoïque acide (2-hydroxy-4,6-diméthoxybenzylidène)-hydrazide,
2,6-difluorobenzoïque acide(2-hydroxy-4,6-diméthoxybenzylidène)-hydrazide,
4-trifluorométhylbenzoïque acide (2-hydroxy-4,6-diméthoxy-benzylidène)-hydrazide, ou
3,4-diméthoxybenzoïque acide (4-diéthylamino-2-hydroxy-benzylidène)-hydrazide.

4. Sel d'un composé selon l'une quelconque des revendications 1 à 3 avec un acide pharmaceutiquement acceptable.

5. Composition pharmaceutique comprenant à titre d'agent actif au moins un composé selon l'une quelconque des revendications 1 à 4.

6. Composition selon la revendication 5, **caractérisée en ce qu'**elle est destinée au traitement et/ou à la prévention des maladies associées à des désordres du métabolisme des lipides.

7. Composition selon l'une des revendications 5 ou 6, **caractérisée en ce qu'**elle est destinée au traitement et/ou à la prévention des maladies cardiovasculaires.

8. Composition selon l'une quelconque des revendications 5 à 7, **caractérisée en ce qu'**elle est destinée au traitement et/ou à la prévention d'une maladie choisie dans le groupe comprenant l'athérosclérose, la resténose artérielle, l'obésité, le diabète de type II, l'ischémie cérébrale, les stéatoses hépatiques, l'hypercholestérolémie, l'hypertriglycéridémie , la dyslipoproteinémie, la chylomicronémie, la lipodistrophie, l'hyperglycémie, l'athérosclérose.

## Claims

1. A compound of the following general formula (I): 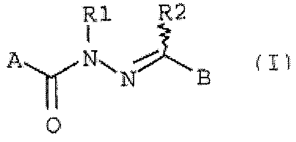 wherein:
- R1 and R2 are selected so as to each be a hydrogen atom,
- A represents a group of the following formula (V): 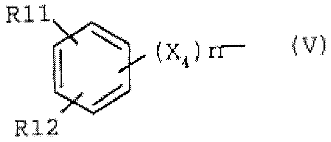
wherein:
- n is 0 or 1
- X₄ if it is present represents a group -CH₂-, or -OCH₂-,-CH=CH,
- R11 and R12, either identical or different, in the ortho, meta or para positions relatively to the bond with -X₄-or relatively to the bond with -CO-when n is zero, are selected from: a lower alkyl group with a linear or branched chain of 1 to 6 carbon atoms or aralkyl group or a fluoroalkyl radical having from 1 to 6 carbon atoms, and from 3 to 7 fluorine atom, an -OH, OR13 radical, wherein R13 represents lower alkyl group with a linear or branched chain of 1 to 6 carbon atoms, a halogen, and more particularly, fluorine and especially in this case, when R11 and R12 are fluorine atoms, they are in the ortho position on either side of the bond with -X₄- or the -CO- radical,
wherein R12 represents a hydrogen atom and R11 represents a sulfonamide group of the SO₂NH₂ type in the para position relatively to the bond with -X₄- or the -CO- radical,
or further R11 represents a hydrogen atom andR12 represents an - Ophenyl group in the ortho position relatively to the bond with -X₄- or the -CO-radical,
- B represents a group of the following formula (II): 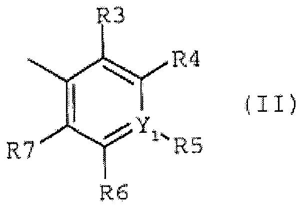
wherein:
- Y₁ is a carbon atom in order to form a phenyl ring or a nitrogen atom for forming a pyridine ring,
- R3 is an -OR8 group wherein R8 represents a linear or branched lower alkyl radical with 1 to 6 carbon atoms, and an amino group -NH₂ or a group - N(r, r') wherein r and r', either identical or different, represent a linear or branched lower alkyl radical, an aryl radical, or a heterocycle wherein r and r' taken together form a heterocycle of variable size, preferably in the para position,
- R4, R5, R6 and R7, either identical or different, are selected from: a hydrogen atom, a halogen atom, and more particularly, fluorine, chlorine and bromine atom, a group of formula -OH, -OR8, or -OCOR9, wherein R8 and R9 represent a linear or branched lower alkyl radical with 1 to 6 carbon atoms, an amino group -NH₂ or a group -N(r, r') wherein r and r', either identical or different, represent a linear or branched lower alkyl radical, an aryl radical, or a heterocycle wherein r and r' taken together form a heterocycle of variable size, preferably in the para position,
at least two of the substituents R4, R5, R6 and R7 being selected as being a hydrogen atom.

2. The compound of formula (I) according to claim 1, **characterized in that** Y₁ is a carbon atom.

3. (4-dimethylamino)-N'-[(1E)-(2-hydroxy-4,8-dimethoxyphenyl) methylene]benzohydrazide,
2-phenyethylbenzoic acid (2-hydroxy-4,6-dimethoxy-benzylidene)-hydrazide,
N-[3-2-hydroxy-4,6-dimethoxy-benzylidene-hydrazinocarbonyl)-phenyl]-propionamide,
(3-chloro-phenoxy)-acetic acid (2-hydroxy-4,6-dimethoxybenzylidene)-hydrazide,
2-phenoxy-benzoic acid (2-hydroxy-4,6-dimethoxy-benzylidene)-hydrazide,
2,6-difluorobenzoic acid (2-hydroxy-4,6-dimethoxy-benzylidene)-hydrazide,
4-trifluoromethylbenzoic acid (2-hydroxy-4,6-dimethoxybenzylidene)-hydrazide,
3,4-dimethoxybenzoic acid (4-diethylamino-2-hydroxybenzylidene)-hydrazide.

4. A salt of a compound according to any of claims 1 to 3, with a pharmaceutically acceptable acid.

5. A pharmaceutical composition comprising as an active agent at least one compound according to any of claims 1 to 4.

6. Composition according to claim 5, **characterized in that** it is intended for treating and/or preventing diseases associated with disorders of lipid metabolism.

7. Composition according to one of claims 5 or 6, **characterized in that** it is intended for treating and/or preventing cardiovascular diseases.

8. Composition according to any of claims 5 to 7, **characterized in that** it is intended for treating and/or preventing a disease selected from the group comprising atherosclerosis, arterial restenosis, obesity, diabetes of type II, brain ischemia, hepatic steatoses, hypercholesterolemia, hypertriglyceridemia, dyslipo-proteinemia, chylomicronemia, lipodystrophia, hyperglycemia, atherosclerosis.

## Patentansprüche

1. Eine Verbindung mit der folgenden allgemeinen Formel (I): 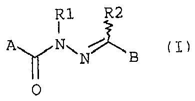 wobei:
- R1 und R2 jeweils als ein Wasserstoffatom ausgewählt sind,
- A eine Gruppe der folgenden Formel (V) darstellt: 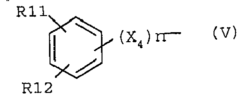
wobei:
- n 0 oder 1 ist,
- X₄, wenn vorhanden, eine Gruppe -CH₂- oder -OCH₂-, -CH=CH-darstellt,
- R11 und R12, identisch oder unterschiedlich, an *ortho-, meta-* oder para-Positionen im Verhältnis zu der Bindung mit -X₄- oder im Verhältnis zu der Bindung mit -CO- wenn n gleich 0, ausgewählt sind aus: einer Niederalkylgruppe mit linearer oder verzweigter Kette mit 1 bis 6 Kohlenstoffatomen oder Aralkyl oder einem Fluoralkylradikal mit 1 bis 6 Kohlenstoffatomen und 3 bis 7 Fluoratomen, einem Radikal -OH-, -OR13-, wobei R13 eine Niederalkylgruppe mit linearer oder verzweigter Kette mit 1 bis 6 Kohlenstoffatomen darstellt, ein Halogen und insbesondere Fluor und ganz speziell in diesem Fall, wenn R11 und R12 Fluoratome sind, sind sie in *ortho-*Position auf der einen und der anderen Seite der Bindung mit -X₄- oder dem Rest -CO-,
- oder R12 ein Wasserstoffatom darstellt und R11 eine Sulfonamidgruppe vom Typ -SO₂NH₂ in para-Position im Verhältnis zu der Bindung mit -X₄- oder dem Rest -CO- darstellt,
oder R11 ferner ein Wasserstoffatom darstellt und R12 eine -Ophenyl-Gruppe in ortho-Position im Verhältnis zu der Bindung mit -X₄- oder dem Rest-CO- darstellt,
- B eine Gruppe der folgenden Formel (II) darstellt: 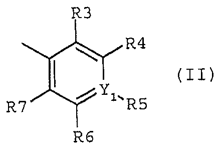
wobei:
- Y₁ ein Kohlenstoffatom ist, um einen Phenylkern zu bilden oder ein Stickstoffatom, um einen Pyridinkern zu bilden,
- R3 eine -OR8-Gruppe ist, wobei R8 ein lineares oder verzweigtes Niederalkylradikal mit 1 bis 6 Kohlenstoffen, eine Aminogruppe -NH₂ oder eine Gruppe -N(r, r') darstellt, in der r und r', identisch oder unterschiedlich, ein lineares oder verzweigtes Niederalkylradikal darstellen, ein Arylradikal, oder eine heterozyklische Verbindung, in der r und r', gemeinsam herangezogen, eine heterozyklische Verbindung variabler Größe bilden, vorzugsweise in *para-*Position,
- R4, R5, R6 und R7, identisch oder unterschiedlich, ausgewählt sind aus: einem Wasserstoffatom, einem Halogenatom und insbesondere aus Fluor, Chlor und Brom, einer Gruppe der Formel -OH, -OR8 oder -OCOR9, wobei R8 und R9 ein lineares oder verzweigtes Niederalkylradikal mit 1 bis 6 Kohlenstoffen, eine Aminogruppe -NH₂ oder eine Gruppe -N(r, r') darstellen, in der r und r', identisch oder unterschiedlich, ein lineares oder verzweigtes Niederalkylradikal, ein Arylradikal oder eine heterozylische Verbindung darstellen, in der r und r', gemeinsam herangezogen, eine heterozyklische Verbindung variabler Größe bilden, vorzugsweise in para-Position,
mindestens zwei der Substituenten R4, R5, R6 und R7 als Wasserstoffatom ausgewählt sind.

2. Verbindung der Formel (I) nach Anspruch 1, **dadurch gekennzeichnet, dass** Y₁ ein Kohlenstoffatom ist.

3. (4-Dimethylamin)-N'-[(1E)-(2-hydroxy-4,6dimethoxyphenyl) methylen]benzohydrazid, 2-Phenethylbenzoesäure (2-hydroxy-4,6-dimethoxy-benzyliden)-hydrazid, N-[3-2-hydroxy-4,6-dimethoxy-benzyliden-hydazincarbonyl)-phenyl]-propionamid, (3-Chlor-phenoxy)-Acetatsäure (2-hydroxy-4,6-dimethoxybenzyliden)-hydrazid, 2-Phenoxybenzoesäure (2-hydroxy-4,6-dimethoxybenzyliden)-hydrazid, 2,6-Difluorbenzoesäure (2-hydroxy-4,6-dimethoxybenzyliden)-hydrazid, 4-Tribluormethylbenzoesäure (2-hydroxy-4,6-dimethoxy-benzyliden)-hydrazid) oder 3,4-Dimethoxybenzoesäure (4-diethylamin-2-hydroxy-benzyliden)-hydrazid.

4. Salz einer Verbindung nach einem der Ansprüche 1 bis 3 mit einer pharmazeutisch akzeptablen Säure.

5. Pharmazeutische Zusammensetzung, die als aktives Mittel mindestens eine Verbindung nach einem der Ansprüche 1 bis 4 umfasst.

6. Zusammensetzung nach Anspruch 5, **dadurch gekennzeichnet, dass** sie zur Behandlung von und/oder Vorbeugung gegen Erkrankungen bestimmt ist, die mit Störungen des Lipidstoffwechsels verbunden sind.

7. Zusammensetzung nach einem der Ansprüche 5 oder 6, **dadurch gekennzeichnet, dass** sie zur Behandlung von und/oder Vorbeugung gegen Herz-Kreislauf-Erkrankungen bestimmt ist.

8. Zusammensetzung nach einem der Ansprüche 5 bis 7, **dadurch gekennzeichnet, dass** sie zur Behandlung von und/oder Vorbeugung gegen eine Erkrankung bestimmt ist, die aus der Gruppe ausgewählt ist, die die Arteriosklerose, die arterielle Restenose, die Obesität, den Diabetes Typ II, die zerebrale Ischämie, die hepatischen Stenosen, die Hypercholesterinämie, die Hypertriglyzeridämie, die Dyslipoproteinämie, die Chylomikronämie, die Lipodistrophie, die Hyperglykämie, die Arteriosklerose umfasst.
